# EUROPEAN PATENT SPECIFICATION

(11) **EP 1 360 578 B1**
(45) Date of publication and mention of the grant of the patent: **19.01.2005**
(21) Application number: 01987061.7
(22) Date of filing: 16.11.2001
(51) Int. Cl.: G06F 3/14, A61B 5/044

(54) **FULLY INTEGRATED CRITICAL CARE WORKSTATION**
VOLLINTEGRIERTER ARBEITSPLATZ FÜR DIE Intensiv-PFLEGE
POSTE DE TRAVAIL ENTIEREMENT INTEGRE POUR SOINS INTENSIFS

(30) Priority: 17.11.2000 US 249572 P
(43) Date of publication of application: 12.11.2003
(73) Proprietor: Draeger Medical Systems, Inc., Danvers, MA 01923 (US)
(72) Inventor: CAVALLARO, Samuel, Warner, NH 03278 (US); SCHOLZ, Wolfgang, Beverly, MA 01915 (US); ORTLAM, Dieter, Salem , MA 01970 (US); ELAZ, Joseph, N. Andover, MA 01845 (US)
(74) Representative: Wilding, Frances Ward
(86) International application number: PCT/US2001/043826
(87) International publication number: WO 2002/041136

(56) References cited:
- WO-A-00/31678
- WO-A-98/59487
- US-A- 4 409 616
- US-A- 5 982 953
- US-A- 6 104 948

## Description

### FIELD OF THE INVENTION

The present invention relates to a critical-care workstation integrating real-time and non-real-time data displays.

### BACKGROUND OF THE INVENTION

In a critical-care environment there are many types of information which a doctor may find important in the treatment of a patient. Currently, each type of information is processed by a separate piece of equipment and displayed on a separate display device. This requires a large amount of space around the patient, and requires the doctor to look at many different display devices to acquire all the information desired.

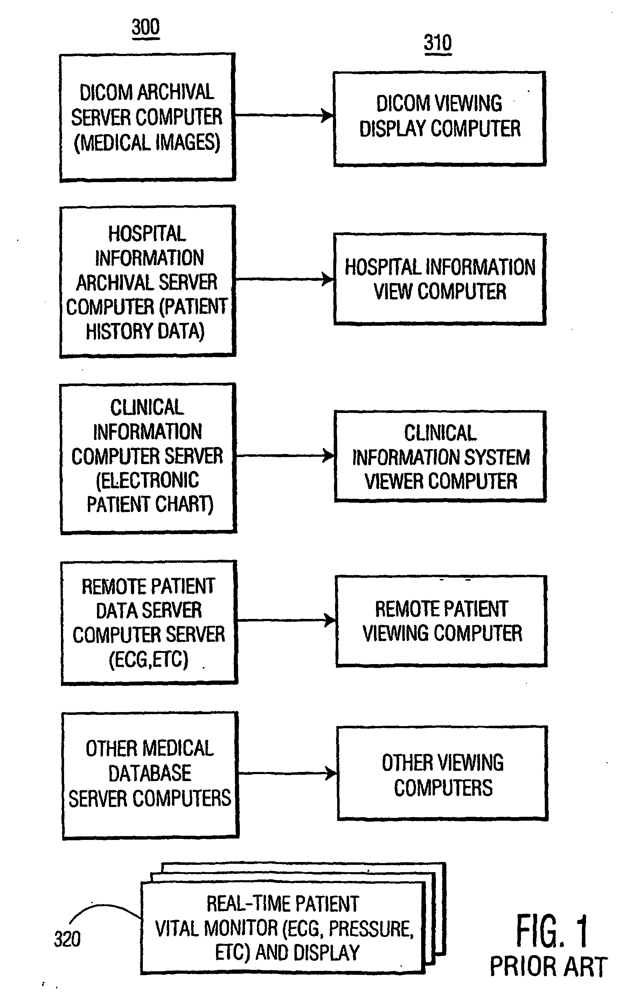

Fig. 1 is a block diagram of an exemplary arrangement of medical devices as just described. In Fig. 1, a plurality 300 of sources of medical information are illustrated. For example, a DICOM archival server computer is a source of medical images, such as X-rays; a hospital information archival server computer is a source of patient history data; and so forth. Each of the plurality 300 of sources includes a corresponding one of a plurality 310 of respective display devices to display the information for the doctor.

For example, the DICOM archival server computer includes a DICOM viewing display computer operating as a client; and the hospital information archival server computer includes a hospital information viewing computer operating as a client; and so forth. Each information server is coupled to corresponding display client via either a direct connection (as illustrated in Fig. 1) or through a network (not shown) in a known manner. The servers 300 and clients 310, in general, store, retrieve and display non-real-time medical information.

The arrangement of Fig. 1 also includes a plurality of sources 320 of real-time medical information, such as electrocardiogram, blood pressure, blood oxygen level, etc. monitors. In Fig. 1, each such monitor includes electrodes (not shown) intended to be connected to the patient, and a monitoring screen (not shown) on which the real-time data collected by the electrodes is displayed. This plurality of equipment, along with the display client computers requires a substantial amount of space in the critical care room, and requires the doctor to look at all the different display devices.

More specifically, medical monitoring systems which display images representing real-time physiological functions of a patient are well known. For example, electrocardiogram (ECG) systems receive signals from electrodes attached to a patient and display waveforms representing patient heart function on a display device. Originally, such systems were implemented in hardwired form, but lately such systems have been implemented by computer systems. These systems include a processor executing a real-time kernel.

Real-time application software operates under control of the real-time kernel to receive the ECG electrode signals and to generate signals conditioning the display device to display an image representing the ECG lead waveforms. Such systems are usually specially designed and implemented systems because the real-time kernels are not in general use. Because of this, they do not include generally available applications, such as image display applications, or internet web browsers, such as are available on more widely used operating systems, e.g. Microsoft Windows.

It has been found, however, that it is often desirable to be able to display both images representing real-time data, such as ECG waveforms, and images representing non-real-time data, such as laboratory results, X-rays, trend data, ventilator loops, etc. One existing system provides two different computer systems, one real-time computer system, such as described above, generating signals representing an image corresponding to the real-time data, and another general-purpose computer system generating signals representing an image corresponding to the non-real-time data. A switch is provided between the two computer systems and the display device, for coupling one of the image representative signals to the display device at a time. In such a system, real-time data is displayed reliably because of the use of the real-time kernel, and display of non-real-time data does not interfere with display of the real-time data because different computer systems are used to control the display of the respective images. However, the doctor may see either the real-time data, or the non-real-time data, but not both simultaneously.

Another existing system is designed to display images representing the real-time data simultaneously with images representing a predetermined set of non-real-time data. For example, such a system may be designed to display ECG images and X-ray images simultaneously. Such a system provides more information to the doctor, but does not permit selection by the doctor of desired non-real-time data. Only the non-real-time data designed into the system can be displayed.

A critical-care display system for a critical care room which provides for the reliable display of real-time data, such as ECG waveforms, simultaneously with selectable non-real-time data from any available source is desirable. The non-real-time data may include images generated by specially programmed medical programs, such as trend data and/or ventilator loop images, or by generally available programs, such as image display programs, word processors, and/or internet browsers.

EP 0668047 discloses an electronic medical device comprising a patient monitoring subsystem which executes on a monitoring CPU and a graphics subsystem which executes on a graphics CPU.

WO 00/46781 discloses an alternate display content controller which provides a parallel graphical user interface adjacent the operating system desktop.

The invention is defined in claim 1 to which reference should now be made. Preferred features are recited in the dependent claims.

### . BRIEF DESCRIPTION OF THE DRAWINGS

In the drawings:
Fig. 1 is a block diagram illustrating the prior art arrangement for displaying real time and non-real time patient-related information;
Fig. 2 is a block diagram illustrating the arrangement for displaying real-time and non-real-time patient-related information according to principles of the present invention;
Fig. 3 is a block diagram of a portion of a critical care workstation according to principles of the present invention; and
Fig. 4 is a block diagram of the software architecture of the software controlling the operation of a critical care workstation according to principles of the present invention.

### DETAILED DESCRIPTION OF THE INVENTION

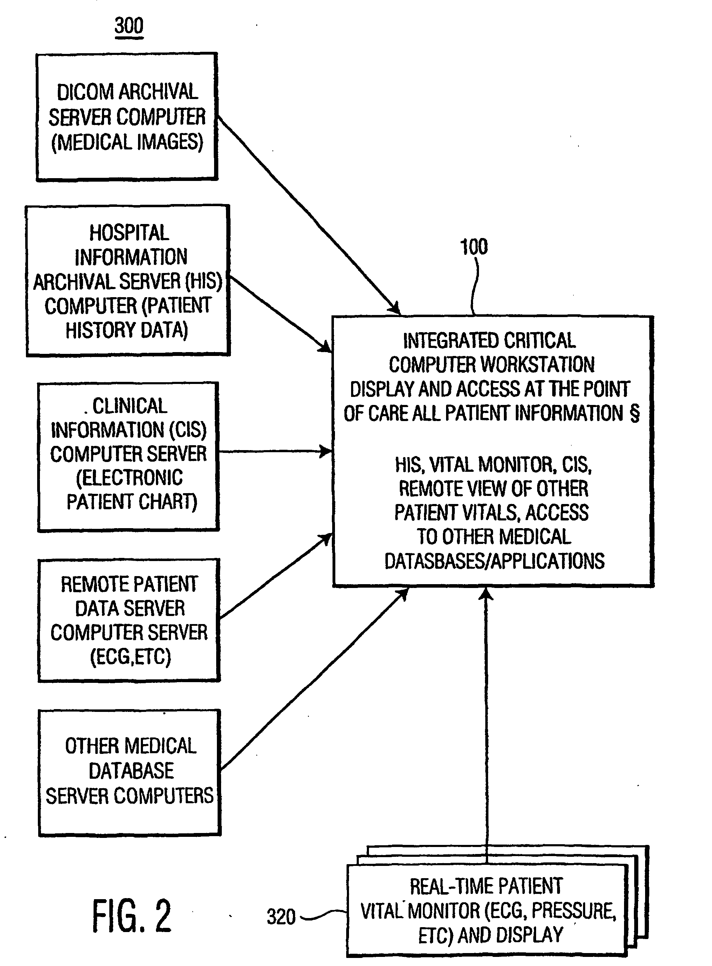

Fig. 2 is a block diagram illustrating a display device which can display real-time and non-real-time patient related information from a plurality of sources concurrently. Those elements in Fig. 2 which are the same as in Fig. 1 are designated by the same reference numbers and are not described in more detail below.

In Fig. 2, the plurality 300 of sources of medical information are connected to an integrated critical computer workstation 100. The workstation 100 receives the medical information from all of the plurality 300 of sources, and displays that information on a single display device. In addition, the real time patient monitors 320 also provide information to the integrated critical care workstation 100, which displays the real time information concurrently with the other, non-real-time patient information, all as described in more detail below.

As with Fig. 1, although Fig. 2 illustrates direct connection between the plurality 300 of medical information sources and the integrated critical care workstation 100, and between the real-time patient monitors 320 and the integrated critical care workstation 100, one skilled in the art will understand that the integrated critical care workstation 100 acts as a client for the server computers 300 and the real-time patient monitors 320, and may be connected to them via a network, such as a local area network. One skilled in the art will further understand that more than a single network may be used to connect the server computers 300 and the real-time monitors 320 to the workstation 100. For example, one or more networks, designed for high performance and a short latency time may be used to connect the real-time monitors 320 to the workstation 100, while one or more slower networks may be used to connect the non-real-time server computers 300 to the workstation 100. However, the details of the connections between the server computers 300 and the workstation 100 and between the real-time monitors 320 and the workstation 100 are not germane to the present invention, and any appropriate connection may be used.

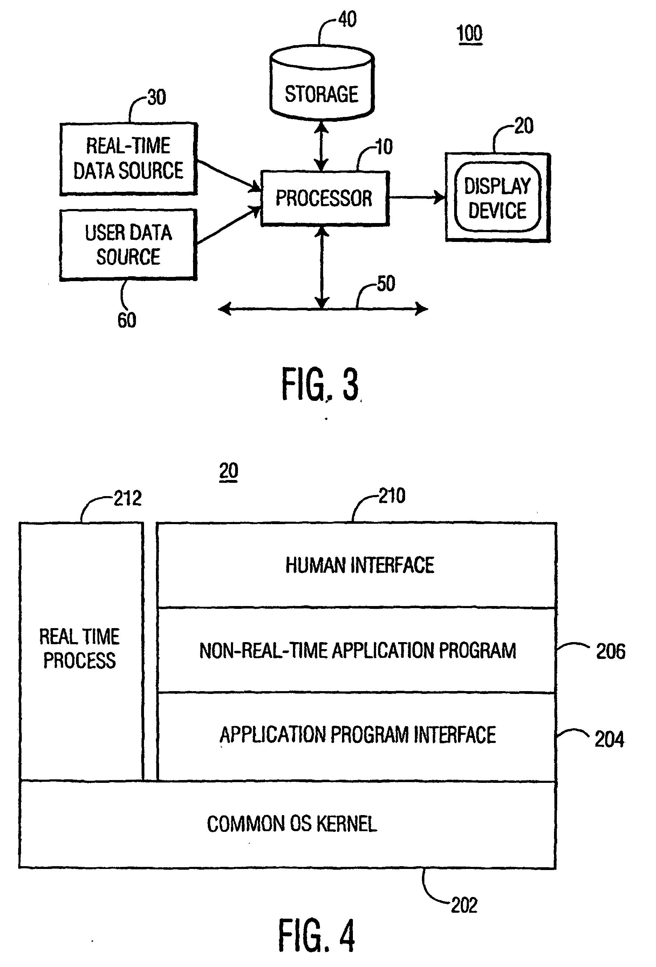

Fig. 3 is a block diagram of a portion of the critical care workstation 100 according to principles of the present invention. In Fig. 3, a processor 10 controls the operation of the critical care workstation 100. An output terminal of the processor 10 is coupled to an input terminal of a display device 20. An output terminal of a source 30 of real-time data, such as, for example, an ECG module, is coupled to a corresponding input terminal of the processor 10. A mass storage device 40 is coupled to the processor 10 via a bi-directional connection. A network connection 50 is also coupled to the processor 10 via a bi-directional connection. Although shown as a single connection, one skilled in the art will understand that the network connection 50 may be in any of the known configurations, e.g. a LAN, and may also include a bridge (not shown) to a wide area network, such as the internet. An output terminal of a source 60 of user input is coupled to an input terminal of the processor 10.

In operation, the real time data source 30, e.g. an ECG module, produces data signals representing, in real-time, the physiological condition of the patient's heart. The processor 10 receives these physiological signals and generates signals representing images corresponding to the physiological signals. The real-time image representative signals are supplied to the display device 20, which displays the images corresponding to the physiological signals. In the illustrated embodiment, the processor 10 executes a real-time kernel. The kernel provides for deterministic execution of a real-time process for receiving the physiological signals from the real-time signal source 30, processing these signals, and generating the image representative signals for the display device 20. For example, for a real-time signal source 30 consisting of an ECG module, signals from the 10 ECG electrodes attached to the patient are received from the real-time signal source 30 and processed by the real-time process in the processor 10 to generate signals representing 12 waveforms corresponding to the 12 lead ECG.

Those signals are supplied to the display device 20 which displays the images of these waveforms. The real-time kernel ensures that waveforms representing the 12 lead ECG are displayed reliably within a predetermined latency time.

Simultaneously with generating signals representing images corresponding to the real-time data, the processor 10 generates image representative signals corresponding to non-real-time data. Images represented by these signals are displayed on the display device 20 simultaneously with the real-time images described above. In the illustrated embodiment the processor 10 executes a generally available windowing operating system, e.g. Microsoft Windows or an Apple Macintosh OS, simultaneously with and independent from the real-time kernel. A non-real-time application program executes under the control of the windowing operating system. Examples of such a non-real-time application program are an internet web browser, a word processor or an image display program.

More specifically, code and data for one or more non-real-time application programs is stored on the storage device 40 or on a server (not shown) on the LAN 50 and/or internet (not shown). A user supplies data to the processor 10 selecting one of the available non-real-time application programs via the user data, source 60. The processor 10 retrieves the code and data for the selected non-real-time application program and executes the application program under control of the windowing operating system. For example, the selected application program may be an image display application which can retrieve data representing an image,
such as an X-ray image from the DICOM archival server computer (of Fig. 1), and produce signals conditioning the display device 20 to display the X-ray image on the display device 20.

Fig. 4 is a block diagram of the software architecture 20 of the software controlling the operation of a critical care workstation according to principles of the present invention. In Fig. 4, a common operating system kernel 202 provides services to programs executing on the processor 10 (of Fig. 3). For example, the common OS kernel 202 provides information relating to available memory, virtual memory, input/output (I/O), etc. The windowing operating system executes as a first process on the processor 10. This is illustrated on the right hand portion of Fig. 4. An application program interface (API) 204 provides a simplified way for a non-real-time application program 206 to access the functions provided by the common OS kernel 202. A human interface layer 210 provides a simplified way for the non-real-time application program 206 to generate display images for the display device 20. The human interface 210 conditions the processor 10 to generate image representative signals in response to the non-real-time application program 206. As described above, these signals are supplied to the display device 20 which displays the image represented by those signals.

A real-time kernel executes as a second process on the processor 10. This is illustrated on the left hand portion of Fig. 4. A real-time process 212 also receives services from the common OS kernel 202.

The real-time kernel in the real-time process 212 provides deterministic execution of the real-time process 212. The real-time process 212, in turn, conditions the processor 10 to receive the real-time signals from the real-time signal source 30, process the real-time signals, and generate image representative signals corresponding to the real time signals. As described above, these signals are also supplied to the display device 20, which displays the image represented by these signals simultaneously with the image represented by the non-real-time signals. One skilled in the art will further understand that the style of the image displayed by the real-time process 212 may be made similar to, or the same as, the style of the image displayed by the non-real-time application program 206 as controlled by the human interface 210.

A system according to Fig. 3 and Fig. 4 allows images corresponding to real-time data to be displayed concurrently with images corresponding to non-real-time data from a plurality of sources. In addition, the non-real-time data may be generated by any application program which may be executed under the control of the windowing operating system. Because a windowing operating system is more commonly available, a wider variety of non-real-time application programs are available to the user and any such program which is made available either on the storage device 40 or on the LAN 50 or internet (not shown) may be selected to be executed.

## Claims

1. A critical care workstation, comprising:
a display device (20);
circuitry, responsive to user input, for selecting a non-real-time display program from among a plurality of available non-real-time display programs; and
a processor (10), coupled to the display device, arranged to execute:
a general purpose operating system for controlling execution of the selected non-real-time application program (206) for displaying images representing non-real-time data on the display device; and
a real-time kernel for controlling execution of a process (212) for monitoring real time data and for displaying images representing the real-time data on the display device simultaneously with the display of the non-real-time data,
**characterized in that** the general purpose operating system and the real-time kernel are both arranged to be executed on the processor as processes using a common operating system kernel (202).

2. The workstation of claim 1 wherein the general purpose operating system is arranged to execute simultaneously with and independently from the real-time kernel.

3. The workstation of claim 1 further comprising a storage device, coupled to the processor, wherein the plurality of available non-real-time application programs are stored on the storage device and the general purpose operating system is arranged to select one of the stored plurality of non-real-time application programs in response to the user input.

4. The workstation of claim 3 wherein the storage device stores code and data representing the non-real-time application program and the processor is arranged to retrieve the stored code and data representing the selected non-real-time application and to control the execution of the retrieved code and data.

5. The workstation of claim 1 further comprising a connection to a network comprising a server capable of storing the plurality of non-real-time application programs, wherein the general purpose operating system is arranged to select one of the stored plurality of non-real-time application programs in response to the user input.

6. The workstation of claim 5 wherein the server stores code and data representing the non-real-time application program and the processor is arranged to retrieve the stored code and data representing the selected non-real-time application and to control the execution of the retrieved code and data.

7. The workstation of claim 1, wherein the real-time data is physiological data.

## Patentansprüche

1. Arbeitsplatz für die Intensivpflege, aufweisend:
eine Displayvorrichtung (20); und eine Schaltung, die auf eine Benutzereingabe anspricht, zum Auswählen eines Nicht-Echtzeit-Displayprogramms unter einer Anzahl an verfügbaren Nicht-Echtzeit-Displayprogrammen;
einen Prozessor (10), der mit der Displayvorrichtung gekoppelt und dafür ausgelegt ist, folgendes zu betreiben:
ein Allzweck-Betriebssystem zum Steuern der Ausführung des ausgewählten Nicht-Echtzeit-Anwendungsprogrammes (206) zum Darstellen von Bildern, welche Nicht-Echtzeitdaten repräsentieren, auf der Displayvorrichtung; und
einen Echtzeitkern zum Steuern der Ausführung eines Prozesses (212) zum Überwachen von Echtzeitdaten und zum Darstellen von Bildern, welche die Echtzeitdaten repräsentieren, auf der Displayvorrichtung gleichzeitig mit der Darstellung der Nicht-Echtzeitdaten,
**dadurch gekennzeichnet, dass** sowohl das Allzweck-Betriebssystem als auch der Echtzeitkern dafür ausgelegt sind, auf dem Prozessor als Prozesse unter Einsatz eines gemeinsamen Betriebssystemkerns (202) ausgeführt zu werden.

2. Arbeitsplatz nach Anspruch 1, wobei das Allzweck-Betriebssystem dafür ausgelegt ist, gleichzeitig mit dem Echtzeitkem und unabhängig von diesem zu arbeiten.

3. Arbeitsplatz nach Anspruch 1, weiter aufweisend eine Speichervorrichtung, die mit dem Prozessor gekoppelt ist, wobei die Anzahl an verfügbaren Nicht-Echtzeit-Anwendungsprogrammen auf der Speichervorrichtung gespeichert ist und das Allzweck-Betriebssystem dafür ausgelegt ist, eines aus der Anzahl der gespeicherten Nicht-Echtzeit-Anwendungsprogrammen als Antwort auf die Benutzereingabe auszuwählen.

4. Arbeitsplatz nach Anspruch 3, wobei die Speichervorrichtung Codes und Daten speichert, welches das Nicht-Echtzeit-Anwendungsprogramm darstellen, und der Prozessor dafür ausgelegt ist, den gespeicherten Code und die Daten, welche die ausgewählte Nicht-Echtzeitanwendung repräsentieren, auszulesen und die Ausführung des ausgeleserien Codes und der ausgelesenen Daten zu steuern.

5. Arbeitsplatz nach Anspruch 1, weiter aufweisend eine Verbindung mit einem Netzwerk, aufweisend einen Server, welcher die Anzahl an Nicht-Echtzeit-Anwendungsprogrammen speichern kann, wobei das Allzweck-Betriebssystem dafür ausgelegt ist, eines aus der Anzahl an gespeicherten Nicht-Echtzeit-Anwendungsprogrammen als Antwort auf die Benutzereingabe auszuwählen.

6. Arbeitsplatz nach Anspruch 5, wobei der Server Codes und Daten speichert, welche das Nicht-Echtzeit-Anwendungsprogramm repräsentieren, und der Prozessor dafür ausgelegt ist, den gespeicherten Code und die gespeicherten Daten auszulesen, welche die ausgewählte Nicht-Echtzeitanwendung repräsentieren, und die Ausführung des ausgeleserien Codes und der ausgelesenen Daten zu steuern.

7. Arbeitsplatz nach Anspruch 1, wobei die Echtzeitdaten physiologische Daten sind.

## Revendications

1. Un poste de travail pour soins intensifs, comprenant :
un dispositif d'affichage (20) ;
un circuit, répondant à une entrée utilisateur, pour sélectionner un programme d'affichage en temps non réel parmi une pluralité de programmes d'affichage en temps non réel disponibles ; et
un organe de traitement (10), couplé au dispositif d'affichage, prévu pour mettre en oeuvre :
un système d'exploitation polyvalent pour commander l'exécution du programme sélectionné d'application en temps non réel (206) pour afficher sur le dispositif d'affichage des images représentant des données en temps non réel ; et
une partie résidente en temps réel pour commander l'exécution d'un processus (212) pour contrôler des données en temps réel et pour afficher sur le dispositif d'affichage des images représentant les données en temps réel simultanément à l'affichage des données en temps non réel,
**caractérisé en ce que** le système d'exploitation polyvalent et la partie résidente en temps réel sont tous deux prévus pour être exécutés sur l'organe de traitement en tant que processus utilisant une partie résidente de système d'exploitation commune (202).

2. Le poste de travail de la revendication 1 dans lequel le système d'exploitation polyvalent est prévu pour exécuter simultanément et indépendamment de la partie résidente en temps réel.

3. Le poste de travail de la revendication 1 comprenant en outre un dispositif de stockage, couplé à l'organe de traitement, dans lequel la pluralité de programmes d'application en temps non réel disponibles sont stockés sur le dispositif de stockage et dans lequel le système d'exploitation polyvalent est prévu pour sélectionner l'un parmi la pluralité stockée de programmes d'application en temps non réel en réponse à l'entrée utilisateur.

4. Le poste de travail de la revendication 3 dans lequel le dispositif de stockage stocke un code et des données représentant le programme d'application en temps non réel et dans lequel l'organe de traitement est prévu pour récupérer le code stocké et les données représentant l'application en temps non réel sélectionnée et pour commander l'exécution du code et des données récupérés.

5. Le poste de travail de la revendication 1 comprenant en outre une liaison à un réseau comprenant un serveur capable de stocker la pluralité de programmes d'application en temps non réel, dans lequel le système d'exploitation polyvalent est prévu pour sélectionner l'un parmi la pluralité stockée de programmes d'application en temps non réel en réponse à l'entrée utilisateur.

6. Le poste de travail de la revendication 5 dans lequel le serveur stocke un code et des données représentant le programme d'application en temps non réel et dans lequel le dispositif de traitement est prévu pour récupérer le code stocké et les données représentant l'application en temps non réel sélectionnée et pour commander l'exécution du code et des données récupérés.

7. Le poste de travail de la revendication 1, dans lequel les données en temps réel sont des données physiologiques.
